(19) Europäisches Patentamt
European Patent Office
Office européen des brevets

(11) Veröffentlichungsnummer: **0 273 245 B1**

(12) # EUROPÄISCHE PATENTSCHRIFT

(45) Veröffentlichungstag der Patentschrift: **24.10.90**

(51) Int. Cl.⁵: **C07C 271/44**, C08F 20/36, A61K 6/08

(21) Anmeldenummer: **87118117.8**

(22) Anmeldetag: **08.12.87**

(54) **(Meth)-Acrylsäure-Derivate von Triisocyanaten und ihre Verwendung.**

(30) Priorität: **18.12.86 DE 3643216**

(43) Veröffentlichungstag der Anmeldung:
**06.07.88 Patentblatt 88/27**

(45) Bekanntmachung des Hinweises auf die Patenterteilung:
**24.10.90 Patentblatt 90/43**

(84) Benannte Vertragsstaaten:
**AT BE CH DE ES FR GB GR IT LI NL SE**

(56) Entgegenhaltungen:
**DE-A- 2 308 036**
**DE-A- 3 117 006**
**GB-A- 2 079 297**

(73) Patentinhaber: **BAYER AG,**
**D-5090 Leverkusen 1 Bayerwerk(DE)**

(72) Erfinder: **Reiners, Jürgen, Dr., Carl-Rumpff-Strasse 57,**
**D-5090 Leverkusen 1(DE)**
Erfinder: **Süling, Carlhans, Dr.,**
**Carl-Leverkus-Strasse 10, D-5068 Odenthal(DE)**
Erfinder: **Podszun, Wolfgang, Dr., Wolfskaul 4,**
**D-5000 Köln 80(DE)**
Erfinder: **Winkel, Jens, Dr., Hahnenweg 6,**
**D-5000 Köln 80(DE)**

**EP 0 273 245 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Acrylsäure- und Methacrylsäurederivate von aliphatischen Triisocyanaten, im folgenden (Meth)-Acrylsäure-Derivate genannt, und ihre Herstellung. Die neuen Verbindungen können als Monomere für die Anwendung im Dentalbereich eingesetzt werden.

Aus der US 3 425 988 sind Polyurethan-polyacrylate bekannt, die als Klebstoffe verwendet werden können.

In JP 81/127 609 werden ebenfalls Polyurethan-polyacrylate beschrieben, die in Kunststoffen oder als Farbstoffkomponenten verwendet werden.

Zahnfüllungsmaterialien, die beispielsweise Bis-(1,3-dimethacryloyloxy-2-propantriyl)-N,N'-hexamethylendicarbamat enthalten sind aus der US 4 394 494 bekannt. Diese Verbindungen zeigen unbefriedigende Abriebeigenschaften.

Es wurden neue (Meth)Acrylsäure-Derivate von aliphatischen Triisocyanaten der Formel (I)

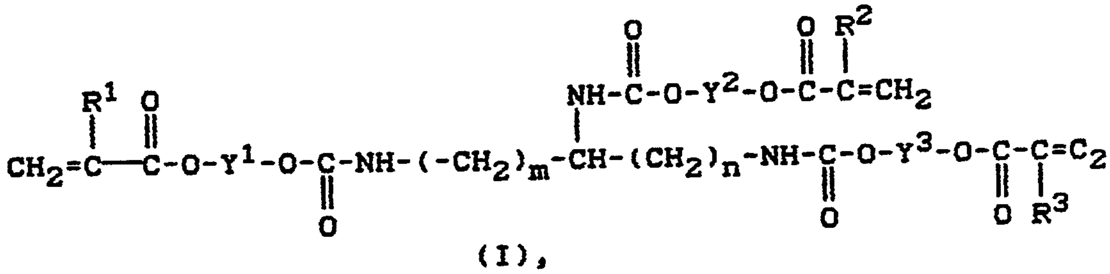

(I),

in der

$R^1$, $R^2$ und $R^3$ gleich oder verschieden sind und Wasserstoff oder Methyl bedeuten,

m und n unabhängig voneinander für eine Zahl von 2 bis 6 stehen,

und

$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)acryloyloxy-Reste substituiert sein können, bedeuten,

gefunden.

Die (Meth)Acrylsäurederivate können als reine Isomere oder als Isomerengemische vorliegen. Für die erfindungs gemäße Verwendung der (Meth)Acrylsäure-Derivate in Dentalmaterialien können Isomerengemische eingesetzt werden.

Im Rahmen der vorliegenden Erfindung können die Substituenten im allgemeinen folgende Bedeutung haben.

Zweiwertige Kohlenwasserstoffreste $Y^1$ bis $Y^3$ können geradkettige oder verzweigte aliphatische Kohlenwasserstoffreste mit 2 bis 15 Kohlenstoffatomen, bevorzugt 2 bis 10 Kohlenstoffatomen, bedeuten. Die Reste $Y^1$ und $Y^3$ können gegebenenfalls 1 bis 3 Sauerstoffbrücken, bevorzugt 1 bis 2 Sauerstoffbrücken, enthalten. Es ist auch möglich, daß die Reste $Y^1$ bis $Y^3$ durch 1 bis 4, bevorzugt 1 bis 2, (Meth)-acryloyloxyreste substituiert sind. Beispielsweise seien die folgenden Reste genannt:

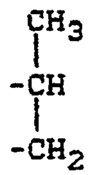 , 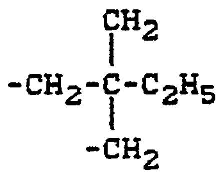 , 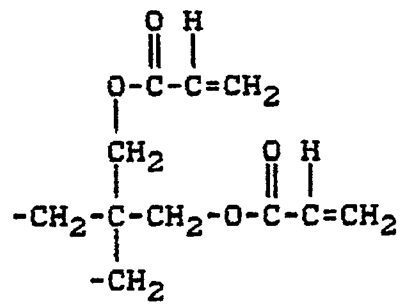 ,

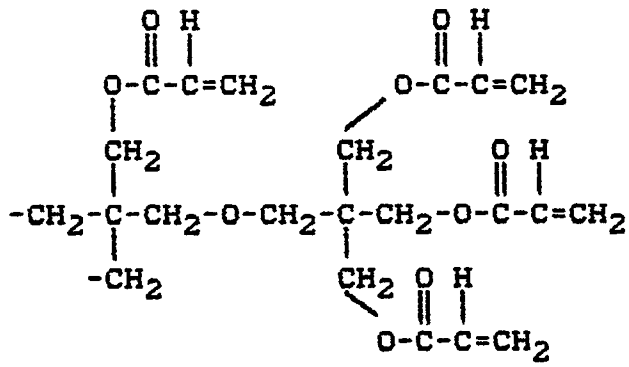 ,

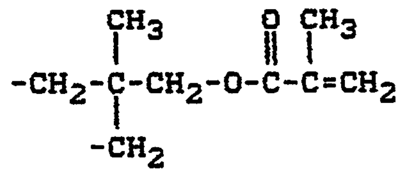

,

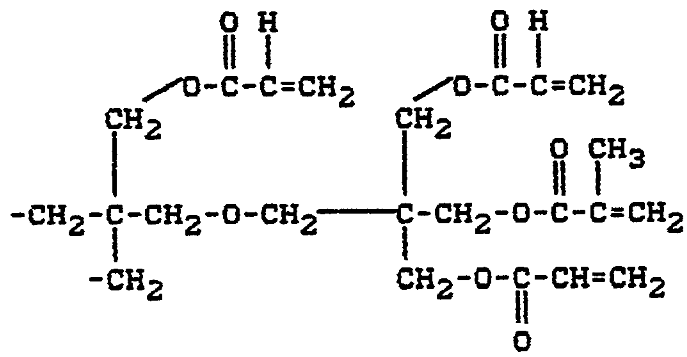

,

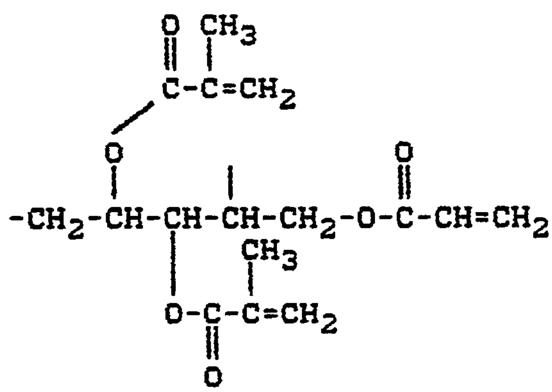

,

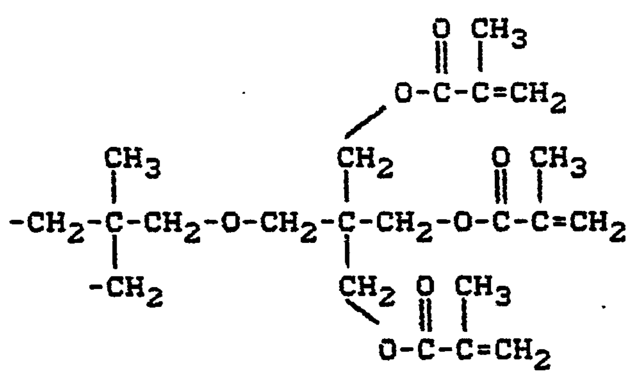

,

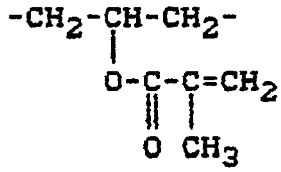 , 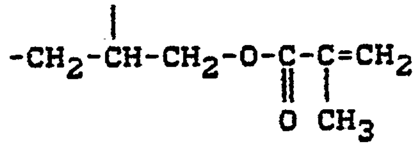 ,

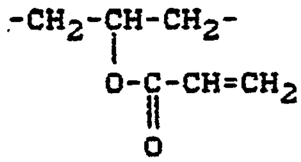 , 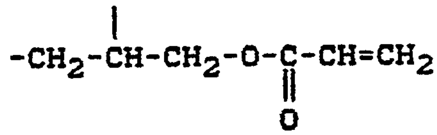

Die neuen (Meth)Acrylsäurederivate sind farblos, schwerflüchtig und ergeben nach der Polymerisation transparente Materialien.

Sie lassen sich besonders gut für Dentalmaterialien, wie Zahnfüllmassen und Beschichtungsmittel, verwenden. Die so erhaltenen Materialien zeichnen sich durch eine überraschend große Widerstandsfähigkeit gegenüber physikalischer und chemischer Beanspruchung aus. In besonderem Maße sind Härte, Abriebfestigkeit und Bruckfestigkeit gegenüber üblichen, zu diesem Zweck eingesetzten Materialien, verbessert.

Bevorzugt sind (Meth)-Acrylsäurederivate der Formel (I)
wobei
$R^1$ bis $R^3$ gleich oder verschieden sind und Wasserstoff oder Methyl bedeuten,
m und n unabhängig voneinander für eine Zahl von 2 bis 6 stehen, wobei die Summe von m + n 8 bis 12 ergibt und
$Y^1$ bis $Y^3$ gleich oder verschieden sind und zweiwertige geradkettige oder verzweigte Kohlenwasserstoffreste mit 2 bis 15 Kohlenstoffatomen, die gegebenenfalls 1 bis 3 Sauerstoffbrücken enthalten können, und gegebenenfalls durch 1 bis 2 zusätzliche (Meth)-acryloyloxy-Reste substituiert sein können, bedeuten.

Insbesondere bevorzugt sind (Meth)Acrylsäurederivate der Formel (I) bei denen
$R^1$ bis $R^3$ gleich oder verschieden sind und Wasserstoff oder Methyl stehen,
m und n gleich oder verschieden sind und für eine Zahl von 2 bis 6 stehen, wobei die Summe von m + n 9 bis 11 ergibt,
und
$Y^1$ bis $Y^3$ gleich oder verschieden sind und zweiwertige geradkettige oder verzweigte aliphatische Kohlenwasserstoffreste mit 2 bis 10 Kohlenstoffatomen, die gegebenenfalls 1 bis 2 Sauerstoffbrücken enthalten können und gegebenenfalls durch 1 bis 2 zusätzliche (Meth)acryloyloxy-Reste substituiert sein können, bedeuten.

Beispielsweise seien die folgenden (Meth)Acrylsäureester-Derivate genannt:

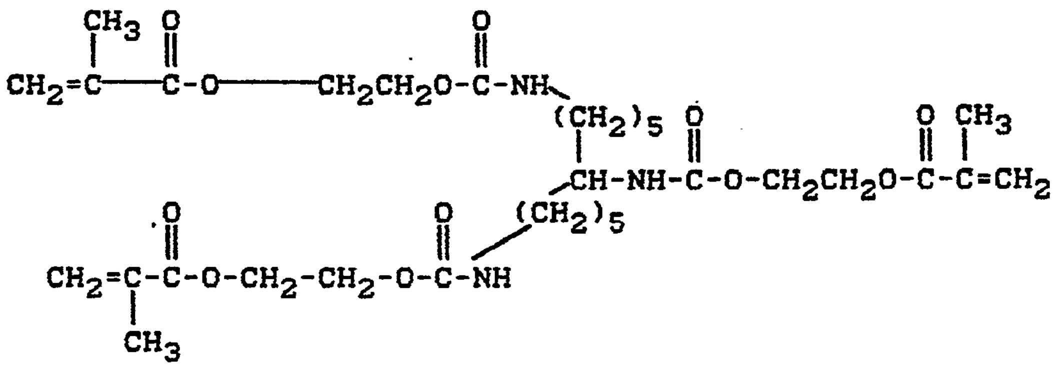

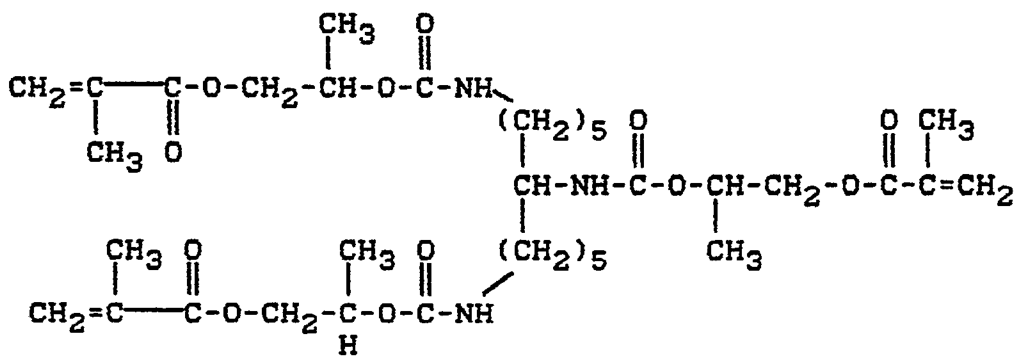

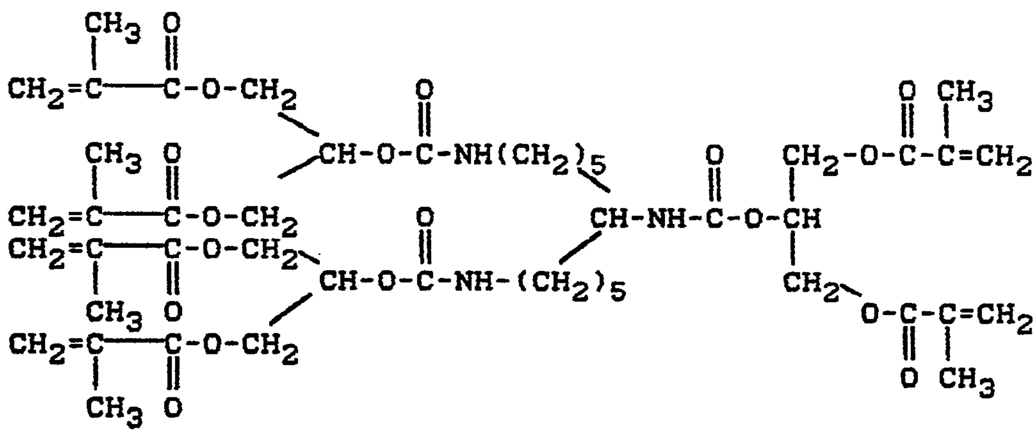

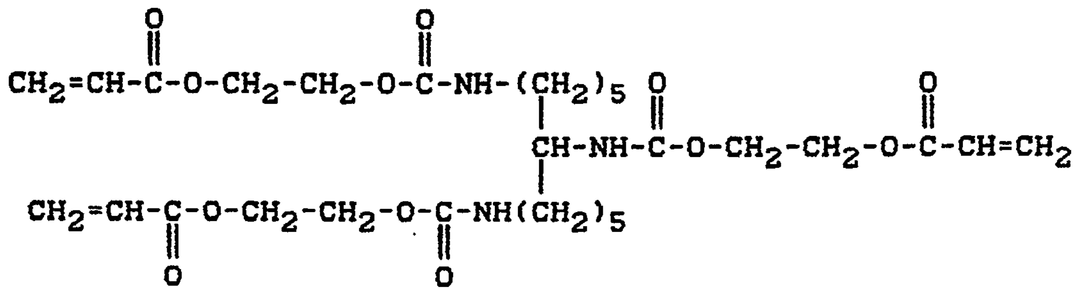

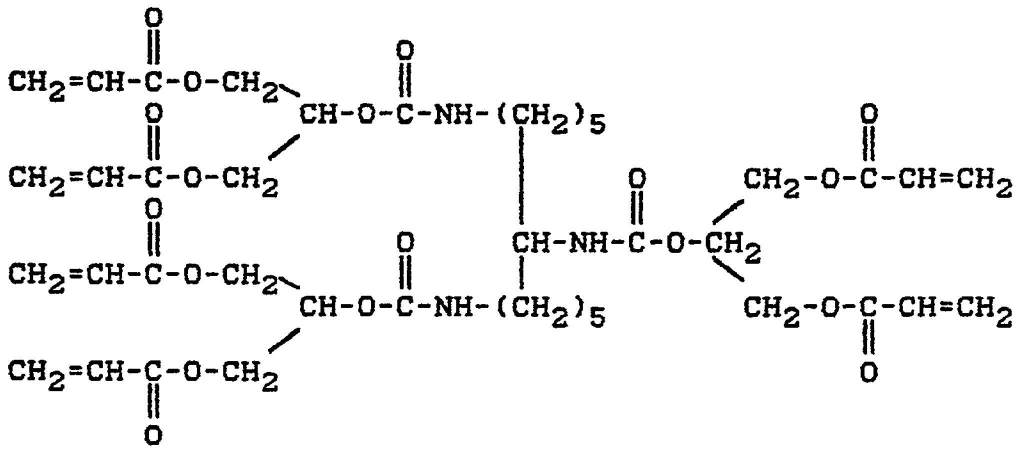

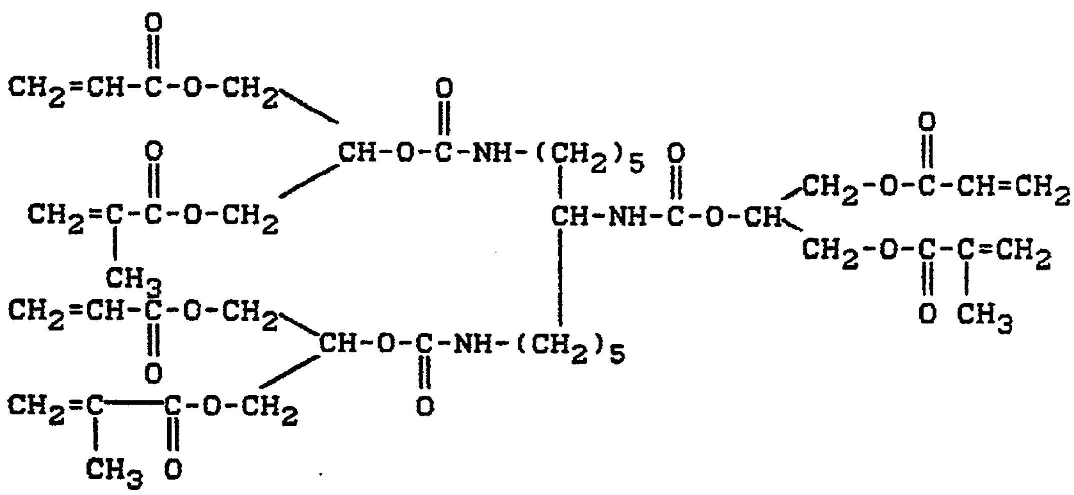

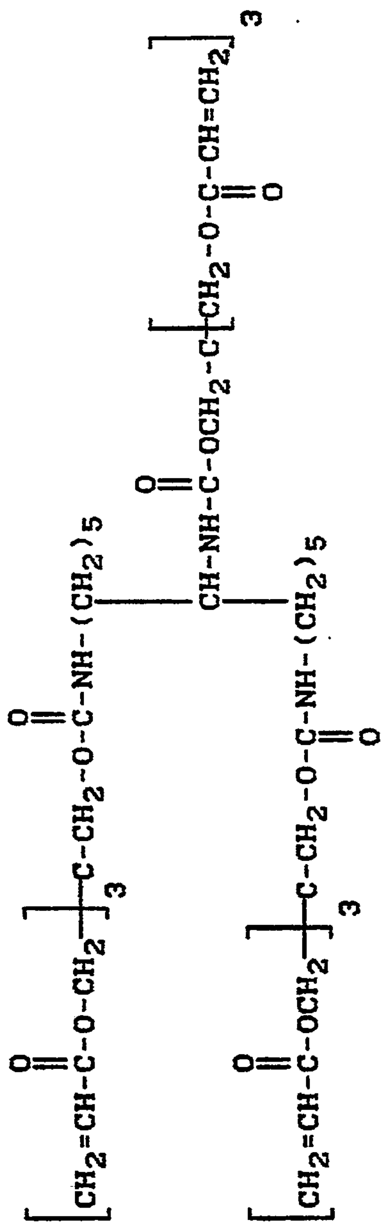

Es wurde auch ein Verfahren zur Herstellung der erfindungsgemäßen (Meth)Acrylsäurederivate gefunden, das dadurch gekennzeichnet ist, daß man ein Triisocyanat der Formel (II)

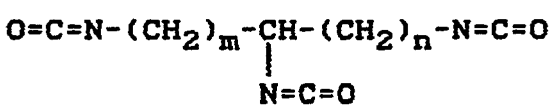

(II),

in der

m und n unabhängig voneinander für eine Zahl von 2 bis 6 stehen,

mit einen Hydroxyalkyl-(Meth)Acrylsäureester der Formel

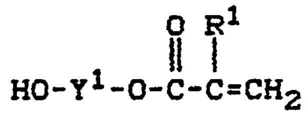 (III),

und/oder

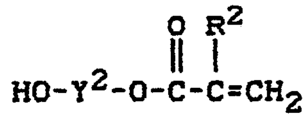 (IV),

und/oder

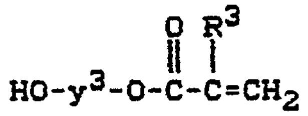 (V),

in denen
$R^1$, $R^2$ und $R^3$ gleich oder verschieden sind und Wasserstoff oder Methyl bedeuten,
und
$Y^1$ bis $Y^3$ gleich oder verschieden sind und zweiwertige geradkettige oder verzweigte Kohlenwasserstoffreste mit 2 bis 15 Kohlenstoffatomen, die gegebenenfalls 1 bis 3 Sauerstoffbrücken enthalten können, und gegebenenfalls durch 1 bis 4 zusätzliche (Meth)-acryloyloxy-Reste substituiert sein können, bedeuten,
umsetzt.

Triisocyanate der Formel II sind bekannt (DE-A 2 924 149) und können durch Phosgenierung der entsprechenden Triaminoverbindungen erhalten werden.

Hydroxyalkyl-(Meth)acrylsäureester der Formel III bis V sind handelsüblich oder können in bekannter Weise durch partielle Veresterung der entsprechenden Polyole hergestellt werden.

Bei der Durchführung des erfindungsgemäßen Verfahrens setzt man im allgemeinen bezogen auf jede Isocyanatgruppe des Triisocyanats der Formel II 0,9 bis 1,1, bevorzugt 1,0 bis 1,05 Mol eines oder mehrerer Hydroxyalkyl-(Meth)Acrylsäureester der Formeln III, IV oder V oder Gemische dieser Verbindungen ein, wobei die Summe aller Hydroxyl-Äquivalente, bezogen auf jede Isocyanatgruppe des Triisocyanats der Formel II, 0,9 bis 1,1, bevorzugt 1,0 bis 1,05, ergeben soll.

Das erfindungsgemäße Verfahren wird im allgemeinen in einem inerten Lösungsmittel durchgeführt. Beispielsweise seien Chloroform, Tetrahydrofuran, Aceton, Dioxan, Dichlormethan, Toluol und Acetonitril genannt. Bevorzugte Lösungsmittel sind Chloroform, Toluol, Aceton und Dichlormethan.

Das erfindungsgemäße Verfahren wird im allgemeinen im Temperaturbereich von 20 bis 100°C, vorzugsweise von 30 bis 70°C, durchgeführt.

Das erfindungsgemäße Verfahren wird im allgemeinen bei Normaldruck durchgeführt. Es ist jedoch auch möglich, das Verfahren im Druckbereich von 1 bis 15 bar durchzuführen.

Bei der erfindungsgemäßen Umsetzung zum Urethan wird vorzugsweise unter Wasserausschluß gearbeitet (vorzugsweise unter 0,1 % Wasser).

Zur Beschleunigung der Umsetzung werden vorzugsweise zinnhaltige Katalysatoren wie Dibutylzinndilaurat, Zinn(II)-octoat oder Dibutylzinndimethoxid verwendet.

Es ist auch möglich, als Katalysatoren Verbindungen mit tertiären Aminogruppen oder Titanverbindungen einzusetzen. Beispielsweise seien die folgenden Katalysatoren genannt: Diazabicyclo[2.2.2]octan, Triethylamin, N-Methylpiperidin, Tetrabutoxy-titan (Ullmann, Encyclopädie der technischen Chemie, Bd. 19, S. 306 (1981)).

Im allgemeinen wird der Katalysator in einer Menge von 0,01 bis 2,5 Gew.-%, bevorzugt von 0,1 bis 1,5 Gew.-% bezogen auf die Gesamtmenge der Reaktanden eingesetzt.

Die Umsetzung zu Urethan wird im allgemeinen in Gegenwart von 0,01 bis 0,2 Gew.-% eines Polymerisationsinhibitors, beispielsweise 2,6-Di-tert.-butyl-4-methylphenol durchgeführt.

Das erfindungsgemäße Verfahren kann beispielsweise wie folgt durchgeführt werden:

Die Reaktanden werden in dem Lösungsmittel gelöst und unter Rühren mit dem Katalysator versetzt. Der zeitliche Verlauf der Umsetzung kann beispielsweise durch Messung der IR-Spektren verfolgt werden. Nach vollständiger Umsetzung der Isocyanatgruppen werden die Reaktionsprodukte durch Entfernen des Lösungsmittels isoliert. Eine vorherige Reinigung mit Hilfe von Absorbentien, bei spielsweise Aktivkohle, Bleicherde, Kieselgel oder Aluminiumoxid ist selbstverständlich auch möglich.

Die erfindungsgemäßen (Meth)-Acrylsäure-Derivate von Triisocyanaten können als Monomere für

polymere Werkstoffe verwendet werden. Die Polymerisation kann in an sich bekannter Weise durch radikalische Initiierung durchgeführt werden und ergibt Polymerisate, die eine hohe Vernetzungsdichte aufweisen.

Die erfindungsgemäßen (Meth)-Acrylsäurederivate von Triisocyanaten können insbesondere als Monomere für Dentalwerkstoffe verwendet werden. Als Dentalwerkstoffe seien beispielsweise Füllungsmaterialien für Zähne, Beschichtungsmittel für Zähne und Komponenten für die Herstellung von Zahnersatz genannt. Je nach Anwendungsgebiet könmen Dentalwerkstoffe weitere Hilfsstoffe enthalten.

Die erfindungsgemäßen Dentalmaterialien enthalten bevorzugt 10 bis 75 Gew.-%, insbesondere bevorzugt 30 bis 70 Gew.-%, an (Meth)Acrylsäure-Derivaten von Triisocyanaten, bezogen auf die eingesetzte Menge der polymerisierbaren Verbindungen.

Für die Anwendung als Monomere für polymere Zahnfüllmassen oder Beschichtungsmittel (Zahnlacke) im Dentalbereich können die erfindungsgemäßen (Meth)-Acrylsäure-Derivate von Triisocyanaten mit an sich bekannten Comonomeren gemischt werden. So kann beispielsweise die Vis kosität dem Verwendungszweck angepaßt werden. Diese Monomermischungen haben im allgemeinen eine Viskosität im Bereich von 60 bis 10.000 mPa.s.

Beispielsweise seien die folgenden Comonomere genannt:
Triethylenglykoldimethacrylat, Tetraethylenglykoldimethacrylat, 1,12-Dodecandioldimethacrylat, 1,6-Hexandioldimethacrylat, Diethylenglykoldimethacrylat, 2,2-Bis[p-(2'-hydroxy-3' methacryloyloxypropoxy)phenyl]propan, 2,2-Bis[p-(2'-methacryloyloxylethoxy)phenyl]propan. Von Vorteil sind auch Comonomere mit Urethangruppen, z. B. die bekannten Umsetzungsprodukte von 1 Mol eines Diisocyanats, z. B. Hexamethylendiisocyanat, Trimethylhexamethylendiisocyanat, Isophorondiisocyanat, mit 2 Mol eines Hydroxyalkyl(meth)acrylsäureesters, z. B. Glycerindimethacrylat und 2-Hydroxypropylacrylat.

Weitere Beispiele für Comonomere sind:
Trimethylolpropan-tri(meth)-acrylat, Bis-(Meth)acryloyloxyethoxymethyl)-tricyclo[5.2.1.0$^{2.6}$]decan (gemäß DE-A 2 931 925 und 2 931 926), 1,3-Di((meth)acryloyloxypropyl)-1,1,3,3-tetramethyl-disiloxan, 1,3-Bis(3-(meth)-acryloyloxyethylcarbamoyloxy-propyl)-1,1,3,3-tetramethyl-disiloxan. Insbesondere werden Comonomere bevorzugt, die bei 13 mbar einen Siedepunkt über 100°C besitzen.

Im Rahmen der vorliegenden Erfindung ist es ebenfalls bevorzugt, Mischungen verschiedener erfindungsgemäßer (Meth)-Acrylsäure-Derivate einzusetzen.

Es ist auch möglich, Monomermischungen einzusetzen, die mehrere Comonomere enthalten.

Die erfindungsgemäßen (Meth)-Acrylsäure-Derivate von Triisocyanaten lassen sich, gegebenenfalls in Mischung mit den genannten Monomeren, mit an sich bekannten Methoden zu vernetzten Polymerisaten aushärten (G.M. Brauer, H. Argentar, Am. Chem. Soc., Symp. Ser. 212, S. 359-371 (1983)). Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind:
Dibenzoylperoxid, Dilauroylperoxid und Di-4-Chlorbenzoylperoxid.

Als tertiäre aromatische Amine seien beispielsweise N,N-Dimethyl-p-toluidin, Bis-(2-hydroxyethyl)-p-toluidin, Bis(2-hydroxyethyl)-3,5-dimethylanilin und das in der DE-A 2 759 239 beschriebene N-Methyl-N-(2-methyl-carbamoyloxypropyl)-3,5-dimethylanilin genannt.

Die Konzentrationen des Peroxids bzw. des Amins werden vorteilhaft so gewählt, daß sie 0,1 bis 5 Gew.-%, bevorzugt 0,5 bis 3 Gew.-%, bezogen auf die Monomermischung betragen. Die peroxid- bzw. aminhaltige Monomermischungen werden bis zur Anwendung getrennt gelagert.

Die erfindungsgemäßen Monomeren können auch durch Bestrahlung mit UV-Licht oder sichtbarem Licht (beispielsweise im Wellenlängenbereich von 230 bis 650 nm) zur Polymerisation gebracht werden. Als Initiatoren für die fotoinitiierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von synergistisch wirkenden Fotoaktivatoren, wie N,N-Dimethylaminoethylmethacrylat, Triethanolamin, 4-N,N-Dimethyl-aminobenzolsulfonsäurebisallylamid.

Die Durchführung des Fotopolymerisationsverfahrens ist beispielsweise in der DE-A 3 135 115 beschrieben.

Neben den oben beschriebenen Initiatoren können den erfindungsgemäßen (Meth)-Acrylsäureestern an sich für diesen Einsatzzweck bekannte Lichtschutzmittel und Polymerisations-Inhibitoren zugesetzt werden.

Das Lichtschutzmittel und der Polymerisations-Inhibitor werden jeweils im allgemeinen in einer Menge von 0,01 bis 0,50 Gew.-Teilen, bezogen auf 100 Gew.-Teile der Monomermischung eingesetzt. Die Monomermischungen können ohne Zusatz von Füllstoffen als Beschichtungsmittel (Zahnlacke) eingesetzt werden.

Bei der Verwendung als Zahnfüllmassen setzt man den erhaltenen Monomermischungen im allgemeinen Füllstoffe zu.

Um einen hohen Füllgrad erreichen zu können, sind Monomermischungen, die eine Viskosität im Bereich von 60 bis 10.000 mPa.s besitzen, besonders vorteilhaft. Den die erfindungsgemäßen Verbindungen der Formel I enthaltenden Monomermischungen können vorzugsweise anorganische Füllstoffe zugemischt werden. Beispielsweise seien Bergkirstall, Quarzit, Kristobalit, Quarzglas, hochdisperse Kieselsäure, Aluminiumoxid und Glaskeramiken, beispielsweise Lanthan und Zirkon enthaltende Glaskeramiken (DE-A 2 347 591) genannt.

Die anorganischen Füllstoffe werden zur Verbesserung des Verbundes zur Polymermatrix des Polymethacrylats vorzugsweise mit einem Haftvermittler vorbehandelt. Die Haftvermittlung kann beispielsweise durch eine Behandlung mit Organosiliciumverbindungen erreicht werden (E.P. Plueddemann, Progress in Organic coatings, 11, 297 bis 308 (1983)). Bevorzugt wird 3-Methacryloyloxypropyltrimethoxysilan eingesetzt.

Die Füllstoffe für die erfindungsgemäßen Zahnfüllmassen weisen im allgemeinen einen mittleren Teilchendurchmesser von 0,01 bis 100 µm, vorzugsweise von 0,05 bis 50 µm auf, besonders bevorzugt 0,05 bis 5 µm. Es kann auch vorteilhaft sein, mehrere Füllstoffe nebeneinander einzusetzen, die einen voneinander verschiedenen Teilchendurchmesser und verschiedenen Silanisierungsgrad besitzen.

Der Füllstoffanteil in den Zahnfüllmassen beträgt im allgemeinen 5 bis 85 Gew.-%, vorzugsweise 50 bis 80 Gew.-%.

Für die Herstellung der Zahnfüllmassen werden die Komponenten unter Verwendung handelsüblicher Knetmaschinen verarbeitet.

Der Anteil der erfindungsgemäßen (Meth)-Acrylsäure-Derivate in den Füllmassen beträgt im allgemeinen 10 bis 75 Gew.-% vorzugsweise 15 bis 60 Gew.-%, bezogen auf die Füllmasse.

Die erfindungsgemäßen (Meth)-Acrylsäure-Derivate von Triisocyanaten können auch als Komponenten bei der Herstellung von Zahnersatz eingesetzt werden.

Dabei werden die erfindungsgemäßen Monomeren mit den üblicherweise verwendeten, an sich bekannten Bestandteilen kombiniert. Vorzugsweise werden die Monomeren im Gemisch mit Alkylmethacrylaten, wie Methylmethacrylat eingesetzt. Es können auch zusätzlich an sich bekannte Perlpolymerisate zugesetzt werden. Zur Einstellung der Zahnfarbe können bekannte anorganische und organische Farbpigmente und Trübungsmittel zugesetzt werden. Auch die Anwendung von Stabilisatoren und Lichtschutzmitteln ist möglich.

Die Kunststoffzähne werden durch radikalische Polymersation der Dentalmassen unter Formgebung hergestellt.

Die Verarbeitung ist sowohl nach Injecktionsverfahren als auch Prägeverfahren möglich und erfolgt im allgemeinen nach den üblichen Herstellungsmethoden für Zähne auf Basis von Poly(methylmethacrylat), z. B. durch thermische Polymerisation unter Verwendung von an sich bekannten Polymerisationsinitiatoren, beispielsweise auf Basis von Peroxiden und Azoverbindungen, wie Dibenzoylperoxid, Dilauroylperoxid, Cyclohexylpercarbonat, Azoisobuttersäuredinitril. Gut geeignet sind auch Mischungen von Polymerisationsinitiatoren mit unterschiedlichen Zerfallstemperaturen.

Die aus den erfindungsgemäßen (Meth)Acrylsäureestern hergestellten Dentalwerkstoffe zeichnen sich durch hohe Widerstandsfähigkeit gegenüber mechanischer Beanspruchung und eine hohe Abrasionsbeständigkeit aus.

Beispiel 1

Herstellung des Adduktes aus 1,6,11-Triisocyanatoundecan und Glyderindimethacrylat

27,9 g (0,1 Mol) 1,6,11-Triisocyanato-undecan werden in 100 ml Chloroform gelöst. Diese Mischung wird mit 48 mg 2,6-Di-tert.-butyl-4-methyl-phenol und 100 mg Zinn-(II)-oktoat versetzt. Bei 30°C werden unter Rühren 68,4 g (0,3 Mol) Glycerindimethacrylat (Isomerengemisch von 1,3- und 1,2-Bis-methacryloyloxy-propanol) zugetropft. Nach beendeter Zugabe wird bei 50 bis 60°C gerührt, bis die Isocyanatgruppen vollständig umgesetzt sind.

Der Umsatz wird durch Messung der IR-Spektren (Isocyanatbande bei ~2200 $cm^{-1}$) verfolgt.

Das Reaktionsgemisch wird abgekühlt, über Celite® filtriert und im Vakuum von Lösungsmittel befreit.

Beispiel 2

Beispiel 1 wird wiederholt, mit der Änderung, daß man nach beendeter Reaktion Triethylenglykoldimethacrylat (kurz: TEGDMA) zum Reaktionsgemisch hinzufügt und anschließend das Lösungsmittel im Vakuum entfernt. Der TEGDMA-Gehalt der zur Gewichtskonstanz eingeengten Monomermischung beträgt 40 Gew.-%.

Beispiel 3

Herstellung einer Zahnfüllmasse

198,3 Gewichtsteile einer Monomermischung aus Beispiel 2, bestehend aus 60 Gew.-% des Adduktes aus 1,6,11-Triisocyanato-undecan und Glycerindimethacrylat und 40 Gew.-% Triethylenglykoldimethacrylat,
0,4 Gewichtsteile Campherchinon,
0,25 Gewichtsteile Benzildimethylketal und
1,0 Gewichtsteile 4-N,N-Dimethylaminobenzoesulfonsäurebisallylamid
werden unter Lichtausschluß zu einer Monomerlösung verarbeitet. Diese härtet durch sichtbares Licht und/oder UV-Licht bei einer Belichtungsdauer von 60 Sekunden zu einem Kunststoff aus, der eine hohe mechanische Stabilität aufweist.

Zur Herstellung einer Zahnfüllmasse werden 38 Gewichtsteile der oben genannten Monomerlösung und 62 Gewichtsteile einer mit 5 Gew.-% 3-Methacryloyloxypropyl-trimethoxysilan silanisierten pyrogenen Kieselsäure (Aerosil OX 50) in einem handelsüblichen Kneter bei Raumtemperatur zu einer Paste verarbeitet. Ein nach DIN 13922 mit einer handelsüblichen Dentallampe ausgehärteter Probekörper, der aus dieser Paste hergestellt wurde, zeigt einen sehr hohen Biegemodul, eine sehr hohe Biegefestigkeit und eine verbesserte Abrasionsbeständigkeit.

## Patentansprüche

1. (Meth)Acrylsäure-Derivate von Triisocyanaten der Formel

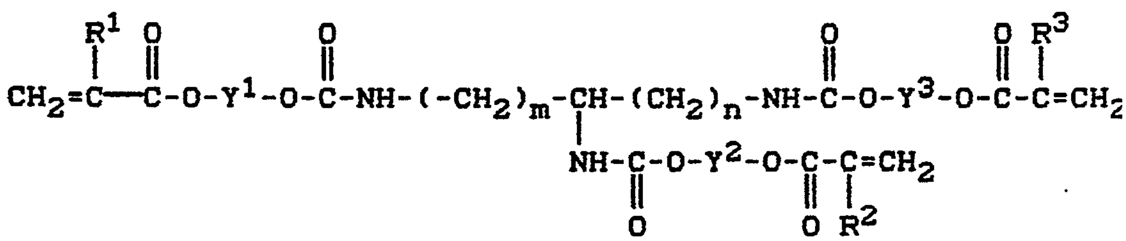

in der
$R^1$, $R^2$ und $R^3$ gleich oder verschieden sind und Wasserstoff oder Methyl bedeuten,
m und n unabhängig voneinander für eine Zahl von 2 bis 6 stehen,
und
$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können, gegebenenfalls durch 1 bis 4 zusätzliche (Meth)-acryloyloxy-Reste substituiert sein können, bedeuten.

2. (Meth)Acrylsäure-Derivate von Triisocyanaten nach Anspruch 1,
wobei
$R^1$, $R^2$ und $R^3$ gleich oder verschieden sind und Wasserstoff oder Methyl bedeuten,
m und n unabhängig voneinander für eine Zahl von 2 bis 6 stehen, wobei die Summe von m + n 8 bis 12 ergibt,
und
$Y^1$ bis $Y^3$ gleich oder verschieden sind und zweiwertige geradkettige oder verzweigte aliphatische Kohlenwasserstoffreste mit 2 bis 10 Kohlenstoffatomen, die gegebenenfalls 1 bis 2 Sauerstoffbrücken enthalten können und gegebenenfalls durch 1 bis 2 zusätzliche (Meth)-acryloyloxy-Reste substituiert sein können, bedeuten.

3. (Meth)Acrylsäure-Derivate von Triisocyanaten nach den Ansprüchen 1 und 2,
wobei
$R^1$, $R^2$ und $R^3$ gleich oder verschieden sind und Wasserstoff oder Methyl bedeuten,
m und n gleich oder verschieden sind und für eine Zahl von 2 bis 6 stehen, wobei die Summe m + n 9 bis 11 ergibt,
$Y^1$ bis $Y^3$ gleich oder verschieden sind und zweiwertige geradkettige oder verzweigte aliphatische Kohlenwasserstoffreste mit 2 bis 10 Kohlenstoffatomen, die gegebenenfalls 1 bis 2 Sauerstoffbrücken enthalten können und gegebenenfalls durch 1 bis 2 zusätzliche (Meth)-acryloyloxy-Reste substituiert sein können, bedeuten.

4. Verfahren zur Herstellung von (Meth)Acrylsäure-Derivaten von Triisocyanaten der Formel

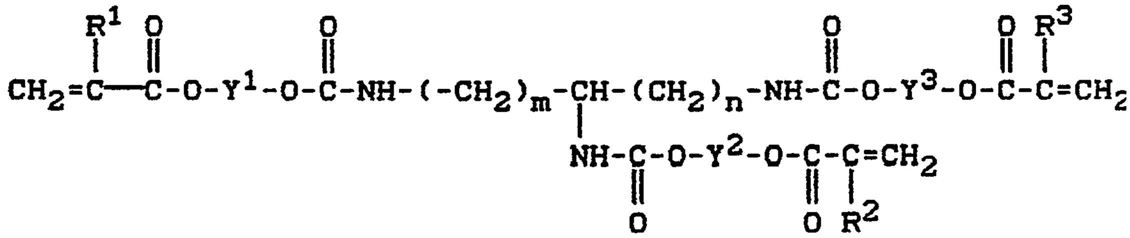

in der
$R^1$, $R^2$ und $R^3$ gleich oder verschieden sind und Wasserstoff oder Methyl bedeuten,
m und n unabhängig voneinander für eine Zahl von 2 bis 6 stehen,
und
$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können, gegebenenfalls durch 1 bis 4 zusätzliche (Meth)-acryloyloxy-Reste substituiert sein können, bedeuten,
dadurch gekennzeichnet, daß man ein Triisocyanat der Formel

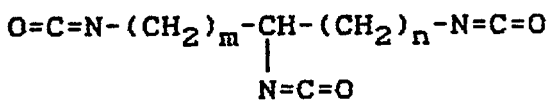 (II),

in der
m und n die obengenannte Bedeutung haben,
mit einen Hydroxyalkyl-(Meth)-Acrylsäureester der Formeln

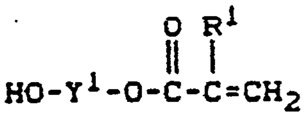 (III),

und/oder

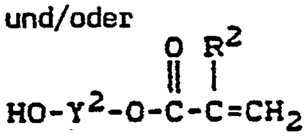 (IV),

und/oder

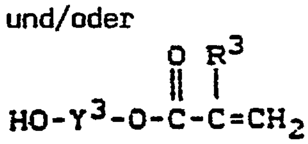 (V),

in denen
$R^1$, $R^2$, $R^3$, $Y^1$ und $Y^3$ die obengenannte Bedeutung haben,
umsetzt.

5. Verfahren nach Anspruch 4, dadurch gekennzeichnet, daß man bezogen auf jede Isocyanatgruppe des Triisocyanats 0,9 bis 1,1 mol einer oder mehrerer der Hydroxyalkyl-(Meth)Acrylsäureester einsetzt.

6. Verfahren nach Ansprüchen 4 und 5, dadurch gekennzeichnet, daß man die Umsetzung im Temperaturbereich von 20 bis 100°C durchführt.

7. Polymerisat aus (Meth)Acrylsäure-Derivaten von Triisocyanaten der Formel

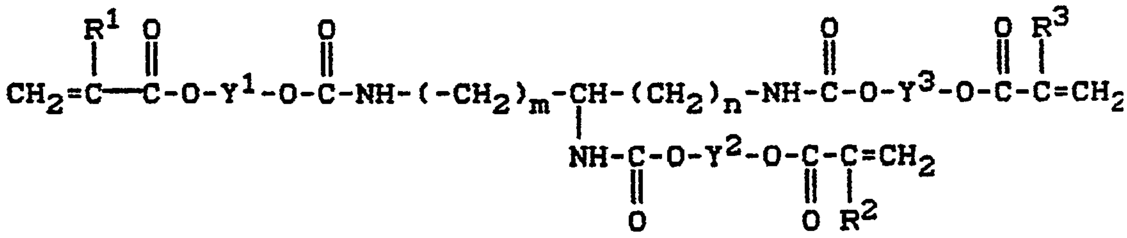

in der
$R^1$, $R^2$ und $R^3$ gleich oder verschieden sind und Wasserstoff oder Methyl bedeuten,
m und n gleich oder verschieden sind und Werte von 2 bis 6 annehmen, und
$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-acryloyloxy-Reste substituiert sein können, bedeuten.

8. Verwendung von (Meth)Acrylsäure-Derivaten von Triisocyanaten der Formel

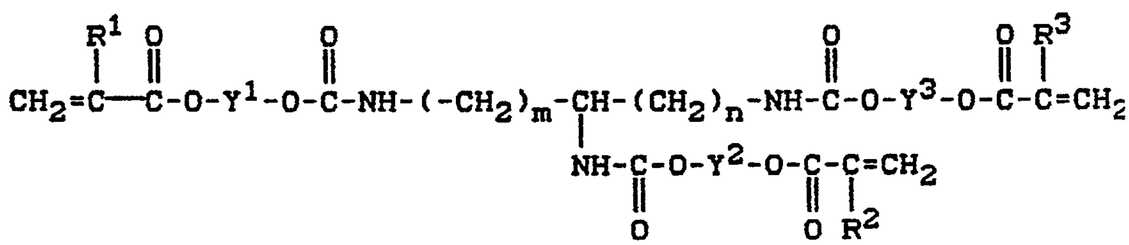

in der
$R^1$, $R^2$ und $R^3$ gleich oder verschieden sind und Wasserstoff oder Methyl bedeuten,
m und n unabhängig voneinander Werte für eine Zahl von 2 bis 6 stehen,
und
$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können, gegebenenfalls durch 1 bis 4 zusätzliche (Meth)-acryloyloxy-Reste substituiert sein können, bedeuten,
im Dentalbereich.

9. Verwendung nach Anspruch 8, dadurch gekennzeichnet, daß die (Meth)Acrylsäure-Derivate von Triisocyanaten in Zahnfüllmassen eingesetzt werden.

10. Verwendung nach Anspruch 8, dadurch gekennzeichnet, daß die (Meth)Acrylsäure-Derivate von Triisocyanaten in Beschichtungsmitteln für Zähne eingesetzt werden.

11. Verwendung nach Anspruch 8, dadurch gekennzeichnet, daß die (Meth)Acrylsäure-Derivate von Triisocyanaten für die Herstellung von Kunststoffzähnen eingesetzt werden.

12. Dentalmaterialien, dadurch gekennzeichnet, daß sie (Meth)Acrylsäure-Derivate von Triisocyanaten der Formel

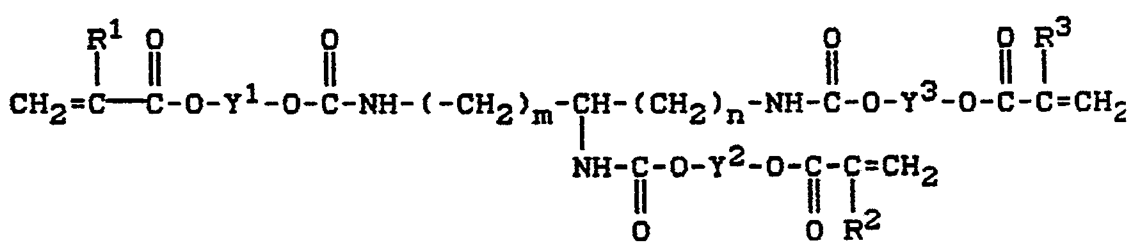

in der
$R^1$, $R^2$ und $R^3$ gleich oder verschieden sind und Wasserstoff oder Methyl bedeuten,
m und n unabhängig voneinander sind für eine Zahl von 2 bis 6 stehen,
und
$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können, gegebenenfalls durch 1 bis 4 zusätzliche (Meth)-acryloyloxy-Reste substituiert sein können, bedeuten,
enthalten.

13. Dentalmaterialien nach Anspruch 12, dadurch gekennzeichnet, daß sie 10 bis 75 Gew.-% an (Meth)Acrylsäure-Derivaten von Triisocyanaten, bezogen auf die eingesetzte Menge der Monomermischung, enthalten.

**Claims**

1. (Meth)acrylic acid derivatives of triisocyanates of the formula

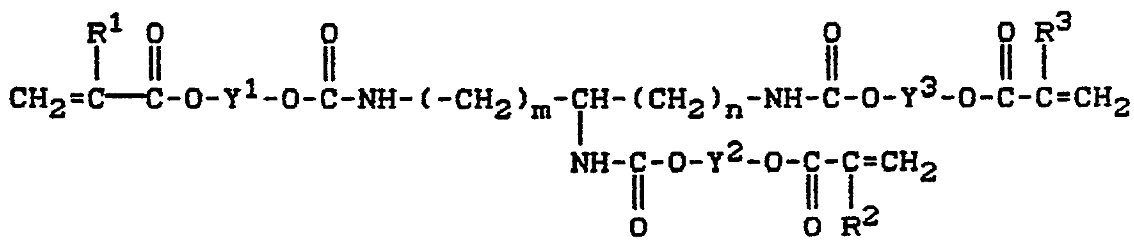

in which

$R^1$, $R^2$ and $R^3$ are identical or different and denote hydrogen or methyl,
m and n independently of one another represent a number from 2 to 6 and
$Y^1$ to $Y^3$ are identical or different and denote divalent straight-chain or branched aliphatic hydrocarbon radicals which have 2 to 15 carbon atoms, can optionally contain 1 to 3 oxygen bridges and can optionally be substituted by 1 to 4 additional (meth)acryloyloxy radicals.

2. (Meth)acrylic acid derivatives of triisocyanates according to Claim 1, wherein
$R^1$, $R^2$ and $R^3$ are identical or different and denote hydrogen or methyl,
m and n independently of one another represent a number from 2 to 6, the sum of m + n being 8 to 12, and
$Y^1$ to $Y^3$ are identical or different and denote divalent straight-chain or branched aliphatic hydrocarbon radicals which have 2 to 10 carbon atoms, can optionally contain 1 to 2 oxygen bridges and can optionally be substituted by 1 or 2 additional (meth)acryloyloxy radicals.

3. (Meth)acrylic acid derivatives of triisocyanates according to Claims 1 and 2, wherein
$R^1$, $R^2$ and $R^3$ are identical or different and denote hydrogen or methyl,
m and n are identical or different and represent a number from 2 to 6, the sum of m + n being 9 to 11, and
$Y^1$ to $Y^3$ are identical or different and denote divalent straight-chain or branched aliphatic hydrocarbon radicals which have 2 to 10 carbon atoms, can optionally contain 1 or 2 oxygen bridges and can optionally be substituted by 1 or 2 additional (meth)acryloyloxy radicals.

4. Process for the preparation of (meth)acrylic acid derivatives of triisocyanates of the formula

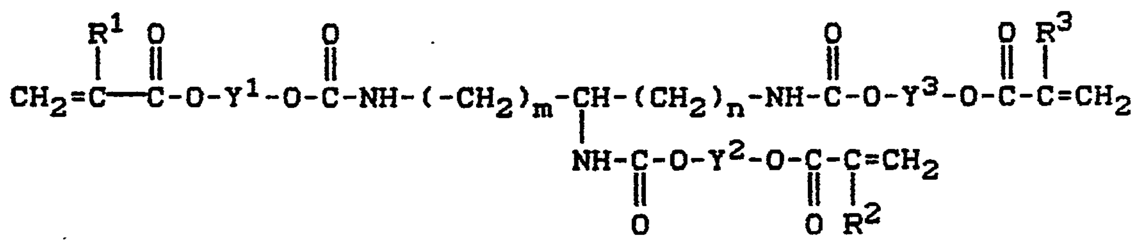

in which

$R^1$, $R^2$ and $R^3$ are identical or different and denote hydrogen or methyl, m and n independently of one another represent a number from 2 to 6 and
$Y^1$ to $Y^3$ are identical or different and denote divalent straight-chain or branched aliphatic hydrocarbon radicals which have 2 to 15 carbon atoms, can optionally contain 1 to 3 oxygen bridges and can optionally be substituted by 1 to 4 additional (meth)acryloyloxy radicals,
characterized in that a triisocyanate of the formula

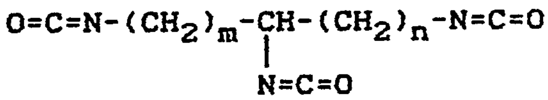

(II),

in which
m and n have the abovementioned meaning, is reacted with a hydroxyalkyl (meth)acrylate of the formulae

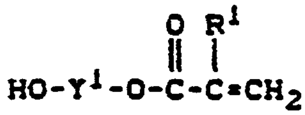

(III),

and/or

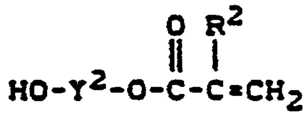

(IV),

and/or

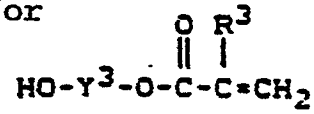

(V),

in which
$R^1$, $R^2$, $R^3$, $Y^1$ and $Y^3$ have the abovementioned meaning.

5. Process according to Claim 4, characterized in that 0.9 to 1.1 mol of one or more of the hydroxyalkyl (meth)acrylates is employed for each isocyanate group of the triisocyanate.

6. Process according to Claims 4 and 5, characterized in that the reaction is carried out in the temperature range from 20 to 100°C.

7. Polymer of (meth)acrylic acid derivatives of triisocyanates of the formula

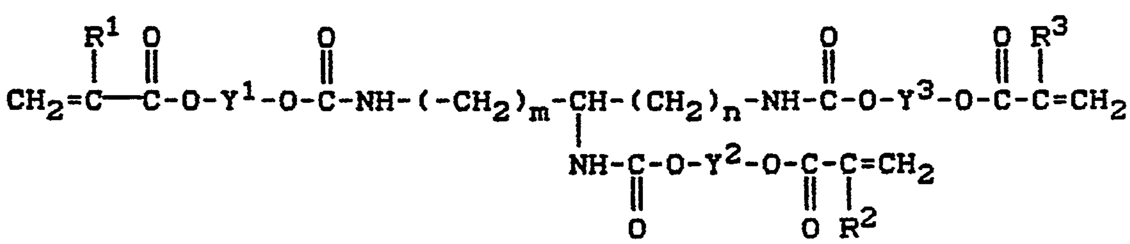

in which
$R^1$, $R^2$ and $R^3$ are identical or different and denote hydrogen or methyl,
m and n are identical or different and assume values from 2 to 6 and
$Y^1$ to $Y^3$ are identical or different and denote divalent straight-chain or branched aliphatic hydrocarbon radicals which have 2 to 15 carbon atoms, can optionally contain 1 to 3 oxygen bridges and can optionally be substituted by 1 to 4 additional (meth)acryloyloxy radicals.

8. Use of (meth)acrylic acid derivatives of triisocyanates of the formula

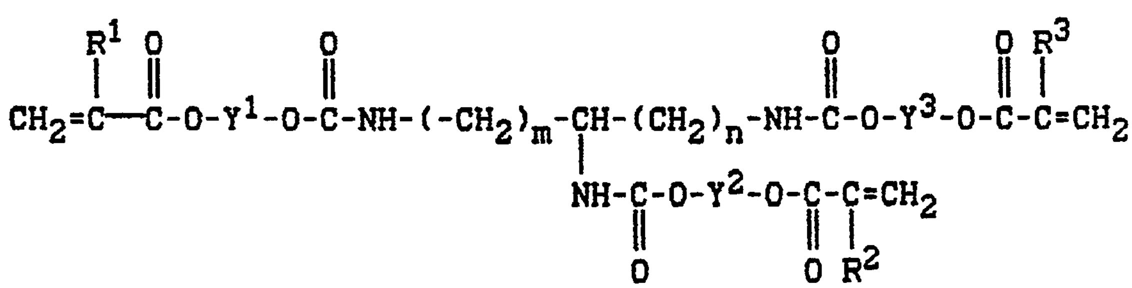

in which
$R^1$, $R^2$ and $R^3$ are identical or different and denote hydrogen or methyl,

m and n independently of one another represent a number from 2 to 6 and
Y[1] to Y[3] are identical or different and denote divalent straight-chain or branched aliphatic hydrocarbon radicals which have 2 to 15 carbon atoms, can optionally contain 1 to 3 oxygen bridges and can optionally be substituted by 1 to 4 additional (meth)acryloyloxy radicals,
in the dental field.

9. Use according to Claim 8, characterized in that the (meth)acrylic acid derivatives of triisocyanates are employed in dental filling compositions.

10. Use according to Claim 8, characterized in that the (meth)acrylic acid derivatives of triisocyanates are employed in coating agents for teeth.

11. Use according to Claim 8, characterized in that the (meth)acrylic acid derivatives of triisocyanates are employed for the production of teeth made of plastic.

12. Dental materials, characterized in that they contain (meth)acrylic acid derivatives of triisocyanates of the formula

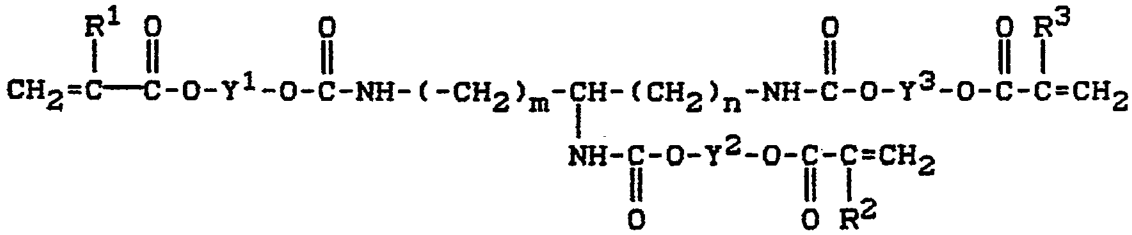

in which
$R^1$, $R^2$ and $R^3$ are identical or different and denote hydrogen or methyl, m and n independently of one another represent a number from 2 to 6 and $Y^1$ and $Y^3$ are identical or different and denote divalent straight-chain or branched aliphatic hydrocarbon radicals which have 2 to 15 carbon atoms, can optionally contain 1 to 3 oxygen bridges and can optionally be substituted by 1 to 4 additional (meth)acryloyloxy radicals.

13. Dental materials according to Claim 12, characterized in that they contain 10 to 75% by weight of (meth)acrylic acid derivatives of triisocyanates, based on the amount of monomer mixture employed.

**Revendications**

1. Dérivés d'acide (méth)acrylique de triisocyanates de formule

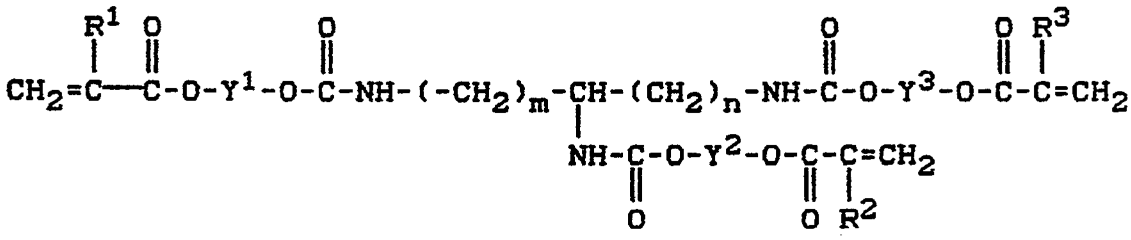

dans laquelle
$R^1$, $R^2$ et $R^3$ sont égaux ou différents et représentent l'hydrogène ou le méthyle,
m et n représentent indépendamment l'un de l'autre un nombre de 2 à 6,
et
$Y^1$ à $Y^3$ sont égaux ou différents et représentent des résidus bivalents d'hydrocarbures aliphatiques à chaîne droite ou ramifiée, comportant 2 à 15 atomes de carbone, qui peuvent renfermer le cas échéant 1 à 3 ponts d'oxygène et être substitués le cas échéant par 1 à 4 résidus (méth)acryloyloxy supplémentaires.

2. Dérivés d'acide (méth)acrylique de triisocyanates selon la revendication 1, dans lesquels
$R^1$, $R^2$ et $R^3$ sont égaux ou différents et représentent l'hydrogène ou le méthyle, met n représentent indépendamment l'un de l'autre un nombre de 2 à 6, la somme m + n étant égale à 8 à 12
et
$Y^1$ à $Y^3$ sont égaux ou différents et représentent des résidus bivalents d'hydrocarbures aliphatiques à chaîne droite ou ramifiée, comportant 2 à 10 atomes de carbone, qui peuvent renfermer le cas échéant 1 à 2 ponts d'oxygène et être substitués le cas échéant par 1 à 2 résidus (méth)acryloyloxy supplémentaires.

3. Dérivés d'acide (méth)acrylique de triisocyanates selon les revendications 1 et 2,
dans lesquels

$R^1$, $R^2$ et $R^3$ sont égaux ou différents et représentent l'hydrogène ou le méthyle,
m et n sont égaux ou différents et représentent un nombre de 2 à 6, la somme m + n étant égale à 9 à 11,
et
$Y^1$ à $Y^3$ sont égaux ou différents et représentent des résidus bivalents d'hydrocarbures aliphatiques à chaîne droite ou ramifiée, comportant 2 à 10 atomes de carbone, qui peuvent renfermer le cas échéant 1 à 2 ponts d'oxygène et être substitués le cas échéant par 1 à 2 résidus méthacryloyloxy supplémentaires.

4. Procédé de fabrication de dérivés d'acide (méth)acrylique de triisocyanates de formule

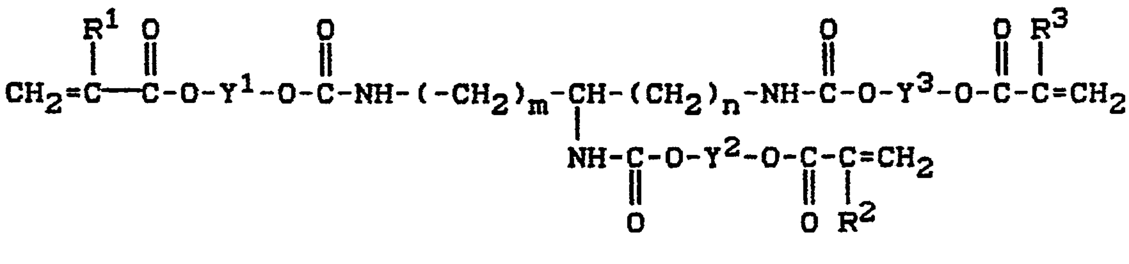

dans laquelle
$R^1$, $R^2$ et $R^3$ sont égaux ou différents et représentent l'hydrogène ou le méthyle,
m et n représentent indépendamment l'un de l'autre un nombre de 2 à 6,
et
$Y^1$ à $Y^3$ sont égaux ou différents et représentent des résidus bivalents d'hydrocarbures aliphatiques à chaîne droite ou ramifiée, comportant 2 à 15 atomes de carbone, qui peuvent renfermer le cas échéant 1 à 3 ponts d'oxygène et être substitués le cas échéant par 1 à 4 résidus (méth)acryloyloxy supplémentaires,
caractérisé en ce que l'on fait réagir un triisocyanate de formule

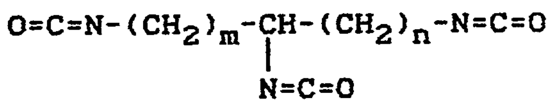
(II),

dans laquelle
m et n possèdent la signification susmentionnée,
avec un ester hydroxyalkylique d'acide (méth)acrylique des formules

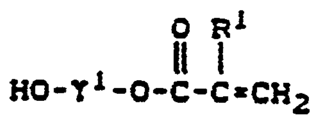
(III),

et/ou

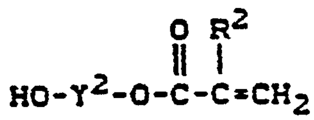
(IV),

et/ou

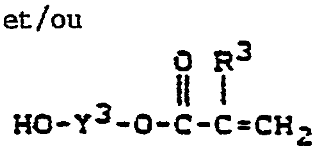
(V),

dans lesquelles
$R^1$, $R^2$, $R^3$, $Y^1$ et $Y^3$ possèdent la valeur susmentionnée.

5. Procédé selon la revendication 4, caractérisé en ce que l'on utilise, par rapport à chaque groupement isocyanate du triisocyanate, 0,9 à 1,1 mole d'un ou de plusieurs esters hydroxyalkyliques d'acide (méth)acrylique.

6. Procédé selon les revendications 4 et 5, caractérisé en ce la réaction est pratiquée dans l'intervalle de température de 20 à 100°C.

7. Polymère des dérivés d'acide (méth)acrylique de triisocyanates de formule

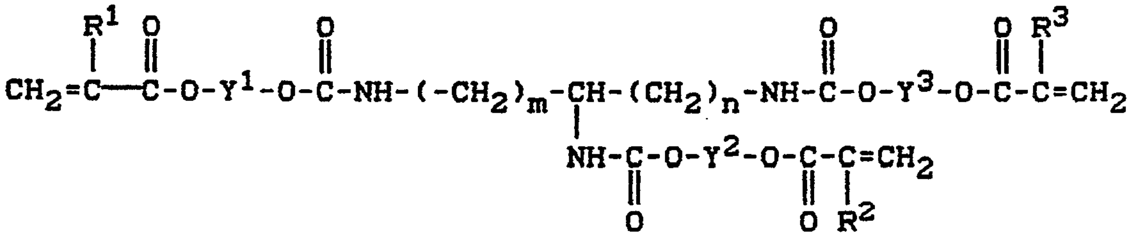

dans laquelle

$R^1$, $R^2$ et $R^3$ sont égaux ou différents et représentent l'hydrogène ou le méthyle,

m et n sont égaux ou différents et représentent un nombre de 2 à 6,

et

$Y^1$ à $Y^3$ sont égaux ou différents et représentent des résidus bivalents d'hydrocarbures aliphatiques à chaîne droite ou ramifiée, comportant 2 à 15 atomes de carbone, qui peuvent renfermer le cas échéant 1 à 3 ponts d'oxygène et être substitués le cas échéant par 1 à 4 résidus (méth)acryloyloxy supplémentaires.

8. Utilisation dans le domaine dentaire de dérivés d'acide (méth)acrylique de triisocyanates de formule

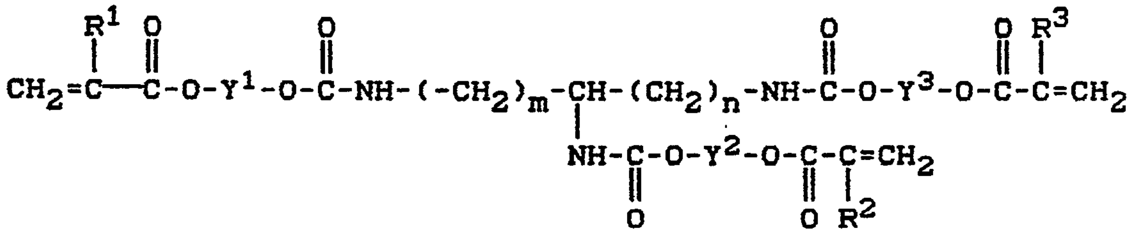

dans laquelle

$R^1$, $R^2$ et $R^3$ sont égaux ou différents et représentent l'hydrogène ou le méthyle,

m et n représentent indépendamment l'un de l'autre un nombre de 2 à 6,

et

$Y^1$ à $Y^3$ sont égaux ou différents et représentent des résidus bivalents d'hydrocarbures aliphatiques à chaîne droite ou ramifiée, comportant 2 à 15 atomes de carbone, qui peuvent renfermer le cas échéant 1 à 3 ponts d'oxygène et être substitués le cas échéant par 1 à 4 résidus (méth)acryloyloxy supplémentaires.

9. Utilisation selon la revendication 8, caractérisée en ce que les dérivés d'acide (méth)acrylique de triisocyanates sont mis en œuvre dans des masses d'obturation des dents.

10. Utilisation selon la revendication 8, caractérisée en ce que les dérivés d'acide (méth)acrylique de triisocyanates sont mis en œuvre dans des agents de revêtement des dents.

11. Utilisation selon la revendication 8, caractérisée en ce que les dérivés d'acide (méth)acrylique de triisocyanates sont mis en œuvre pour la fabrication de prothèses dentaires en matière synthétique.

12. Matériaux dentaires, caractérisés en ce qu'ils renferment des dérivés d'acide (méth)acrylique de triisocyanates de formule

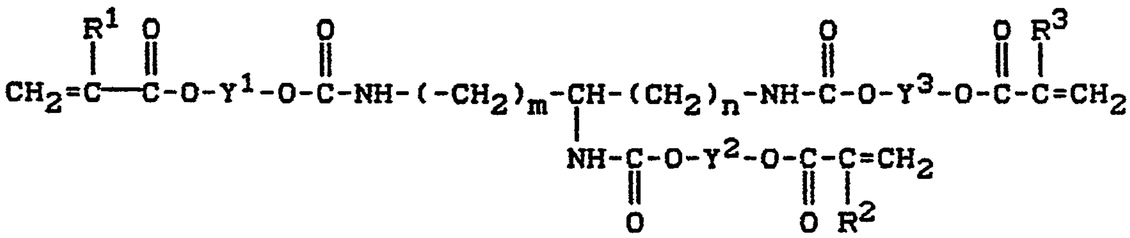

dans laquelle

$R^1$, $R^2$ et $R^3$ sont égaux ou différents et représentent l'hydrogène ou le méthyle,

m et n représentent indépendamment l'un de l'autre un nombre de 2 à 6,

et

$Y^1$ à $Y^3$ sont égaux ou différents et représentent des résidus bivalents d'hydrocarbures aliphatiques à chaîne droite ou ramifiée, comportant 2 à 15 atomes de carbone, qui peuvent renfermer le cas échéant 1 à 3 ponts d'oxygène et être substitués le cas échéant par 1 à 4 résidus (méth)acryloyloxy supplémentaires.

13. Matériaux dentaires selon la revendication 12, caractérisés en ce qu'ils renferment 10 à 75% en poids de dérivés d'acide (méth)acrylique de triisocyanates par rapport à la quantité de mélange de monomères utilisée.